# FASCICULE DE BREVET EUROPEEN

(11) **EP 2 295 440 B1**
(45) Date de publication et mention de la délivrance du brevet: **22.03.2017**
(21) Numéro de dépôt: 10290391.1
(22) Date de dépôt: 13.07.2010
(51) Int. Cl.: C07F 9/165, C08B 1/00

(54) **Nouveaux liquides ioniques à base d'anions thiophosphate et leurs préprations**
Auf Thiophosphatanionen basierende neuartige ionische Flüssigkeiten, und ihre Herstellung
New ionic liquids based on thiophosphate anions and preparations thereof

(30) Priorité: 31.07.2009 FR 0903810
(43) Date de publication de la demande: 16.03.2011
(73) Titulaire: IFP Énergies nouvelles, 92852 Rueil-Malmaison Cedex (FR)
(72) Inventeur: Delfort, Bruno, 75005 Paris (FR); Morvan, Didier, 69440 Mornant (FR); Vallee, Christophe, 38360 Sassenage (FR); Olivier-Bourbigou, Hélène, 69230 Saint Genis-Laval (FR); Pellier, Emmanuel, 69420 Tupin Semons (FR)

(56) Documents cités:
- WO-A1-2008/090155
- WO-A2-2007/138256
- US-A- 5 641 888
- US-B2- 6 824 599
- AKINTONWA D. A. A.: "The synthesis and stereochemical aspects of optically active phosphorothionates" TETRAHEDRON, vol. 34, 1978, pages 959-967, XP002563940
- ENCHEV D ET AL: "2,5-dihydro-1,2-oxaphospholene derivatives by the reaction of 1,2-alkadienephosphonates and phosphorus pseudohalogens" PHOSPHORUS, SULFUR AND SILICON AND THE RELATED ELEMENTS, GORDON AND BREACH SCIENCE PUBLISHERS, AMSTERDAM, GB, vol. 57, no. 3-4, 1 janvier 1991 (1991-01-01), pages 249-253, XP009128006 ISSN: 1042-6507
- JENTZSCH R ET AL: "Quantitative Untersuchungen ueber das Alkylierungsvremoegen kernsubstituierter O,O-dimethyl-o-phenyl-phosphate und -thiophosphate" JOURNAL FUER PRAKTISCHE CHEMIE, WILEY VCH, WEINHEIM, vol. 317, 1 janvier 1975 (1975-01-01), pages 721-732, XP009099637 ISSN: 1436-9966
- HILGETAG G., LEHMAN G., MARTINI A., SCHRAMM G., TEICHMANN H.: "Über alkylierende esterspaltungen einiger Dialkyl-aryl- und Alkyl-diaryl-thiophosphate" JOURNAL FÜR PRAKTISCHE CHEMIE, vol. 8, no. 3-4, 1959, pages 207-223, XP002563941

## Description

### Domaine de l'invention

L'invention porte sur une nouvelle famille de liquides ioniques comprenant un anion de type thiophosphate.

### Étude de l'art antérieur

Les liquides ioniques, de formule générale Q⁺A⁻, sont des sels liquides à des températures inférieures ou égales à 100°C qui présentent des propriétés physico-chimiques particulières. Ils se caractérisent notamment par de faibles ou inexistantes tensions de vapeur et par de grandes plages de stabilité thermique où ils restent liquides. Ces propriétés en font des solvants alternatifs plus respectueux de l'environnement que les solvants organiques volatiles conventionnels, ils permettent notamment d'éviter toute perte de matière pendant l'évaporation et ainsi de réduire l'émission de composés organiques volatils.

Les liquides ioniques sont composés d'ions et ne contiennent pas de molécules neutres, ce qui permet d'obtenir des milieux hautement polaires. Ces propriétés ont fait des liquides ioniques des composés de plus en plus utilisés comme solvants et catalyseurs pour les réactions organiques, catalytiques ou enzymatiques, comme solvants pour les séparations liquide-liquide, les extractions ou encore pour la synthèse de nouveaux matériaux (voir par exemple H. Olivier-Bourbigou, L. Magna, J. Mol. Catal. A, Chem. 2002, 182, 419 "Ionic liquids: perspectives for organic and catalytic reactions"; P. Wasserscheidt Angew. Chem. Int. Ed. Engl. 2000, 39, 3772 "Ionic liquid- Novel solutions for transition-metal catalysis" ; P. Wasserscheidt Ionic Liquids in Synthesis, 2002, Wiley-VCH; R. Sheldon Chem. Comm. 2001, 2399 "Catalytic reactions in ionic liquids" ; M. J. Earle, K. R. Seddon Pure Appl. Chem. 2000, 72, 1391 "Ionic liquids, green solvent for the future"; T. Welton Chem. Rev. 1999, 92, 2071 "Room temperature ionic liquids. Solvents for synthesis and catalysis" R. Hagiwara, Y. Ito J. Fluorine Chem. 2000, 105, 221-227 "Room temperature ionic liquids of alkylimidazolium cations and fluoroanions").

Les liquides ioniques peuvent également être utilisés comme solvant de biopolymères, comme la cellulose ou la biomasse lignocellulosique, comme décrit dans le brevet US-6,824,599. La demande de brevet WO 2007/138256 décrit l'hydrolyse de la cellulose dans un liquide ionique. On peut également citer la demande de brevet WO 2008/090155 qui divulgue de façon générique un liquide ionique de formule [A]⁺ₙ[Y]ⁿ⁻, pour le prétraitement d'un matériau cellulosique au moyen dudit liquide ionique, suivi d'une hydrolyse enzymatique du matériau cellulosique prétraité.

L'engouement pour cette nouvelle classe de solvants résulte de leurs propriétés physico-chimiques qu'il est possible de moduler en changeant la nature de l'anion et du cation. Ainsi, la polarité, l'hydrophobicité ou la lipophilicité, la viscosité peuvent être contrôlées par les choix judicieux des constituants du liquide ionique.

De part toutes ces raisons évoquées, il est donc nécessaire de développer des nouvelles familles de liquides ioniques afin d'adapter les propriétés de ces liquides ioniques à chaque application. C'est dans ce cadre que s'inscrit la présente invention.

### Résumé de l'invention

La présente invention porte sur de nouveaux liquides ioniques à base d'anions thiophosphate et de cations organiques. Leurs procédés de préparation, ainsi que leur utilisation pour la solubilisation et/ou le traitement des polymères sont également décrits.

### Description détaillée de l'invention

Il est décrit ci-après un liquide ionique associant un cation organique Q+ à un anion thiophosphate, répondant à l'une des formules suivantes : 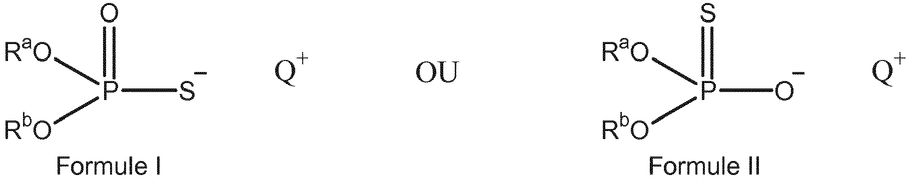 dans lesquelles
- R^{a} et R^{b}, identiques ou différents, représentent des radicaux hydrocarbyles ayant de 1 à 30 atomes de carbone,
   de préférence des groupements alkyles, saturés ou non saturés, cycloalkyles ou aromatiques, aryles ou aralkyles, éventuellement substitués,
- tout ou partie des hydrogènes des radicaux R^{a} et R^{b} pouvant être remplacés par des hétéroatomes, tels que des halogènes,
- les radicaux R^{a} et R^{b} portant éventuellement une ou plusieurs fonctions choisies parmi les fonctions -CO₂R, -C(O)R, -OR, -C(O)NRR', -C(O)N(R)NR'R", -NRR', -SR,-S(O)R, -S(O)₂R, -SO₃R, -CN, -N(R)P(O)R'R', -PRR', -P(O)RR', -P(OR)(OR'),-P(O)(OR)(OR') dans lesquelles R, R' et R", identiques ou différents, représentent chacun l'hydrogène ou des radicaux hydrocarbyles ayant de 1 à 30 atomes de carbone,
le cation Q+ étant choisi parmi
, les cations ammonium quaternaires dérivés d'hétérocycles azotés comportant 1, 2 ou 3 atomes d'azote, de formules générales : 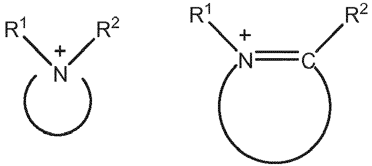 dans lesquelles les cycles sont constitués de 4 à 10 atomes de carbone, de préférence de 5 à 6 atomes de carbone.

Le cation Q⁺, selon la présente invention est choisi dans le groupe formé par le *N-*butylpyridinium, le *N*-éthylpyridinium, l'éthyl-3-méthyl-1-imidazolium, le butyl-3-méthyl-1-imidazolium, l'allyl-3-méthyl-1-imidazolium, le vinyl-3-méthyl-1-imidazolium, le vinyl-3-allyl-1-imidazolium, l'hexyl-3-méthyl-1-imidazolium, le butyl-3-diméthyl-1,2-imidazolium, le cation (hydroxy-2-éthyl)-1-méthyl-3-imidazolium, le cation (carboxy-2-éthyl)-1-méthyl-3-imidazolium, le diéthylpyrazolium, le *N*-butyl-*N*-méthylpyrrolidinium, le *N*-butyl-*N*-méthylmorpholinium, le tétrabutylphosphonium, le tributyl-tétradécyl-phosphonium.

Les radicaux R^{a} et R^{b} peuvent également être liés et représentent alors un unique groupement alkylidène ou phénylidène.

Les anions selon la présente invention sont de préférence choisis parmi les structures suivantes:

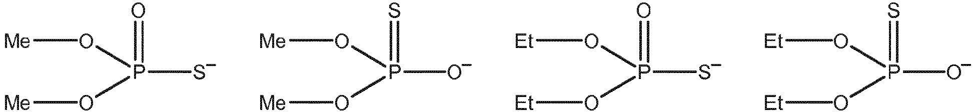

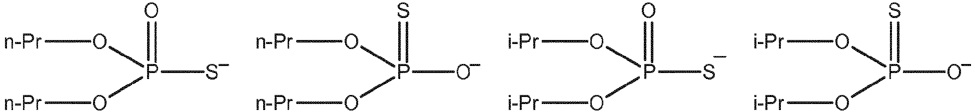

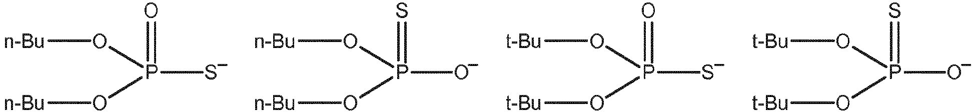

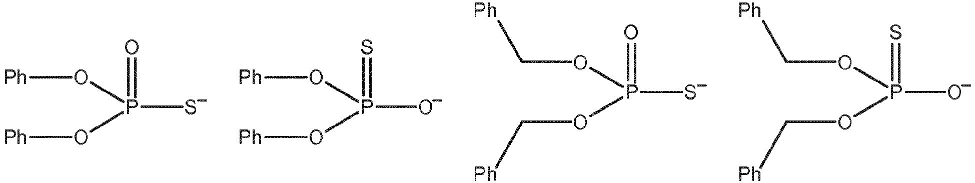

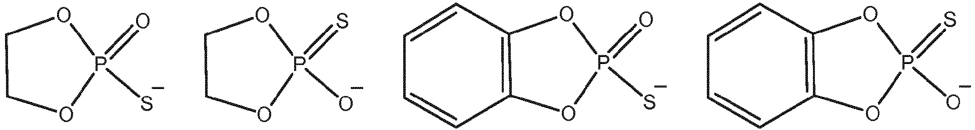

À titre d'exemple de liquide ionique selon la présente invention, on peut citer le diéthylthiophosphate d'allyl-3-méthyl-1-imidazolium, le diéthylthiophosphate de vinyl-3-méthyl-1-imidazolium, le diéthylthiophosphate d'éthyl-3-méthyl-1-imidazolium, le diéthylthiophosphate de butyl-3-méthyl-1-imidazolium, le diphénylthiophosphate d'allyl-3-méthyl-1-imidazolium, le diphénylthiophosphate de vinyl-3-méthyl-1-imidazolium, le diphénylthiophosphate d'éthyl-3-méthyl-1-imidazolium, le diphénylthiophosphate de butyl-3-méthyl-1-imidazolium, le dibenzylthiophosphate d'allyl-3-méthyl-1-imidazolium, le dibenzylthiophosphate de vinyl-3-méthyl-1-imidazolium, le dibenzylthiophosphate d'éthyl-3-méthyl-1-imidazolium, le dibenzylthiophosphate de butyl-3-méthyl-1-imidazolium.

Ces liquides ioniques peuvent être utilisés seuls ou en mélange.

Il est également décrit ci après des procédés de préparation de liquides ioniques associant un cation organique Q⁺ à un anion thiophosphate, répondant à l'une des formules suivantes : 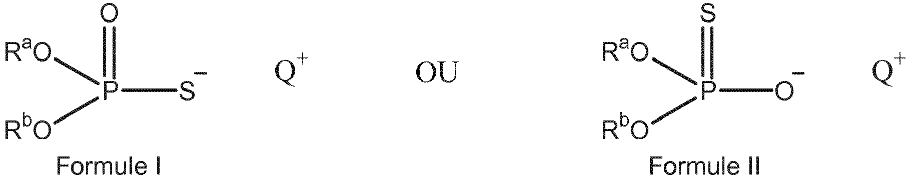 dans lesquelles R^{a} et R^{b}, identiques ou différents, représentent des radicaux hydrocarbyles ayant de 1 à 30 atomes de carbone, tels que définis précédemment. Selon un premier mode de préparation, le liquide ionique selon la présente invention est préparé par une réaction de métathèse d'ions entre :
- un sel de formule générale Q⁺A⁻, avec Q⁺ un cation tel que défini précédemment et A⁻ un anion, organique ou inorganique, et
- un sel de thiophosphate de formule générale: 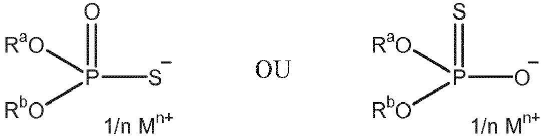 dans laquelle R^{a} et R^{b} sont définis comme précédemment et Mⁿ⁺ est un cation alcalin ou alcalino-terreux et n= 1 ou 2.

De façon préférée, l'anion A⁻ est choisi parmi les anions chlorure, bromure, iodure, nitrate, formate ou acétate. De façon très préférée l'anion est choisi parmi les anions chlorure ou bromure.

De façon préférée, le cation Mⁿ⁺ est choisi parmi les cations lithium, sodium, potassium, magnésium ou calcium. De façon très préférée, le cation Mⁿ⁺ est le cation sodium.

En général, le sel de thiophosphate et le sel de formule générale Q⁺A⁻ sont présents en quantité stoechiométrique dans la réaction. Cependant dans de nombreux cas, il est préférable d'utiliser un excès molaire du sel de thiophosphate par rapport à la quantité de cation introduite, par exemple dans la plage de 1.00 à 2 équivalents. Le manipulateur expérimenté pourra apprécier la nécessité ou non de faire varier les conditions de réaction et de quantité de matière introduite pour améliorer le rendement en liquide ionique.

Selon un autre mode de préparation, le liquide ionique est synthétisé par la réaction entre :
- un sel de formule générale Q⁺A⁻, avec Q⁺ un cation tel que défini précédemment et A⁻ un anion, organique ou inorganique, et
- un acide de formule générale: 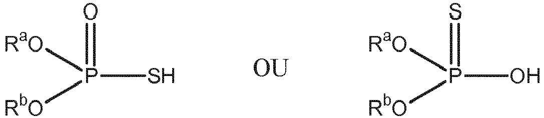 dans laquelle R^{a} et R^{b} sont définis comme précédemment.

De façon préférée, l'anion A⁻ sera choisi parmi les anions alkylcarbonate, de façon préférée méthylcarbonate, acétate, formate, hydroxyde.

En général, l'acide de formule générale: 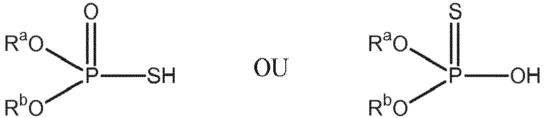 et le sel de formule générale Q⁺A⁻ sont présents en quantité stoechiométrique dans la réaction. Cependant dans de nombreux cas, il est préférable d'utiliser un excès molaire d'acide par rapport à la quantité de cation introduite, par exemple dans la plage de 1.00 à 2 équivalents. Le manipulateur expérimenté pourra apprécier la nécessité ou non de faire varier les conditions de réaction et de quantité de matière introduite pour améliorer le rendement en liquide ionique.

La synthèse des liquides ioniques précédemment décrits peut être réalisée dans une plage de température comprise entre -78°C et 150°C, de façon préférée entre -20°C et 80°C.

Elle peut être effectuée à l'air ou sous une atmosphère de gaz inerte (azote, argon). De façon, préférée, la synthèse est réalisée sous une atmosphère de gaz inerte.

La synthèse des liquides ioniques précédemment décrits peut être réalisée en l'absence de solvant ou dans un solvant organique. Les solvants organiques pour la synthèse des liquides ioniques précédemment décrits peuvent être choisis parmi les hydrocarbures (pentane, hexane, heptane, cyclohexane), les solvants aromatiques (benzène, toluène, xylène), les solvants chlorés (chloroforme, dichlorométhane), les solvants oxygénés (diéthyléther, THF, méthyl-THF, dioxane, diméthoxyéthane), les solvants azotés (acétonitrile, DMF, NMP, pyridine).

Les liquides ioniques synthétisés peuvent être séparés du milieu réactionnel et/ou purifiés selon les méthodes classiques de la chimie organique. De préférence, les liquides ioniques seront séparés du milieu réactionnel par évaporation, par filtration, ou par une combinaison de ces méthodes.

Les liquides ioniques précédemment décrits peuvent être utilisés comme solvants ou catalyseurs.

Les liquides ioniques selon la présente invention peuvent être utilisés dans des applications variées, par exemple comme solvant de réaction en chimie organique ou pour les réactions enzymatiques, comme matrice pour des techniques analytiques telles que la spectrométrie de masse MALDI (matrix assisted laser désorption ionisation), comme solvant pour des procédés d'extraction, comme vecteur en chromatographie, comme lubrifiant ou comme fluide hydraulique. Les liquides ioniques peuvent également être utilisés dans le domaine de la catalyse, des piles à combustible, de l'électrochimie, de la solubilisation et de la transformation des biomatériaux.

De façon préférée, les liquides ioniques selon la présente invention sont utilisés pour la solubilisation et/ou le traitement des polymères, de façon préférée des biopolymères tel que la biomasse lignocellulosique, la cellulose et d'une façon plus générale les matériaux riches en cellulose.

Plus particulièrement, les liquides ioniques précédemment décrits peuvent servir à solubiliser et à convertir la cellulose en de nombreux produits chimiques. La solubilisation permet notamment la fonctionnalisation de la cellulose comme l'estérification ou l'éthérification qui conduisent à une grande variété de matériaux. Ainsi, les esters de cellulose trouvent de nombreuses applications dans l'industrie du papier, pour la préparation de fibres et de textiles ou encore comme polymères ou films.

La solubilisation de la cellulose dans les liquides ioniques selon la présente invention permet la fonctionnalisation, par exemple, par des groupements acétyles, tosyles ou trityles.

Les liquides ioniques revendiqués peuvent également être utilisés comme prétraitement de la biomasse lignocellulosique et ainsi améliorer l'étape associée d'hydrolyse enzymatique dans le schéma général de production de bioéthanol.

Les liquides ioniques selon la présente invention peuvent également servir dans la production de bioproduits et biocarburants issus de matériaux riches en cellulose, tel que le 5-hydroxyméthylfurfural (HMF), le sorbitol, le diméthylfurane, l'acide lévulinique ou encore les esters et éthers d'HMF.

Enfin, l'utilisation des liquides ioniques selon l'invention dans le traitement de la biomasse lignocellulosique peut permettre la séparation et la valorisation de la lignine présente dans ces matériaux en produits à valeurs ajoutées (résines, colles, bioplastiques, polyester...) et comme source de biocarburant (après pyrolyse).

Les exemples ci-après illustrent l'invention sans en limiter la portée.

### Exemples

### Exemple 1: Synthèse de l'allyl-3-méthyl-1-imidazolium diéthylthiophosphate (AMI-DEPS):

Sous argon, dans une solution de 15,64 g (98,6 mmol) d'allyl-3-méthyl-1-imidazolium chlorure dans 150 ml de dichlorométhane anhydre, on ajoute 20,96 g (98,6 mmol) de diéthylthiophosphate de sodium. La suspension est agitée 2 heures à température ambiante. 20 ml d'ether diéthylique sont ensuite ajoutés et la solution est filtrée avec une canule sous argon (papier Whatman n°5). Le filtrat est ensuite évaporé sous vide. On obtient 26,06 g d'un liquide marron-orangé, le rendement est de 90 %.

¹H RMN (300 MHz, DMSO): δ 9,48 (s, 1 H, N-C*H*-N), 7,78 (s, 1 H, N-CH-CH-N), 7,77 (s, 1 H, N-CH-CH-N), 6,05 (dt, 1 H, CH₂-C*H*-CH₂), 5,34 (m, 2H, CH-C*H*₂), 4,9 (m, 2H, C*H*₂-CH), 3,9 (s, 3H, N-C*H*₃), 3,66 (qt, 2H, C*H*₂-CH₃), 1,07 (t, 3H, CH₂-C*H*₃). ³¹P{H} RMN (300 MHz, DMSO): δ 53,1. ¹³C{H} RMN (300 MHz, DMSO): δ 137 (N-CH-N), 131,9 (*C*H-CH₂), 123,7 (CH-*C*H₂), 122,2-120 (N-CH-CH-N), 59,4 (O-*C*H₂), 50,6 (N-*C*H₂-CH), 35,7 (N-*C*H₃), 16,3 (O-CH₂-*C*H₃).

### Exemple 2: Solubilisation de la cellulose:

19 g de liquide ionique AMI-DEPS et 1 g de cellulose Avicel PH 101 (Sigma-Aldrich) sont placés dans un ballon de 170 ml et sont agités mécaniquement par des pâles à 70°C pendant 1 h. La cellulose est complètement soluble dans ces conditions.

### Exemple 3: Prétraitement et hydrolyse enzymatique:

19 g de liquide ionique AMI-DEPS et 1 g de cellulose Avicel PH 101 (Sigma-Aldrich) sont placés dans un ballon de 170 ml et sont agités mécaniquement par des pâles à 70°C pendant 1 h dans un agitateur Tornado^{©} muni d'un carrousel 6 positions (Radleys).
Après ce prétraitement, le chauffage est coupé et 80 ml d'eau distillée sont ensuite rapidement ajoutés au mélange : la cellulose précipite. La suspension contenant le liquide ionique, l'eau et la cellulose est placée dans un tube à centrifugation et agitée à 9500 tr/min pendant 10 minutes. Le surnageant (ou eau de lavage) contenant le liquide ionique est ensuite éliminé. L'opération est répétée trois fois par addition d'eau distillée sur la partie solide encore présente dans le tube à centrifugation.
La cellulose dite amorphe récupérée est ensuite utilisée en hydrolyse enzymatique. Le solide récupéré après les lavages à la centrifugeuse est placé dans des flacons Schott de 100 ml. On ajoute 5 ml de tampon acétate, 10 ml d'une solution 1% poids de NaN₃ dans de l'eau puis on complète jusqu'à 100 g avec de l'eau distillée. Cette solution est ensuite laissée une nuit à 50°C pour "activation" sous une agitation de 550 tr/min dans un bain marie à sec STEM. On ajoute ensuite à la solution des quantités connues d'enzyme:
- Cellulases XL508, 10FPU par gramme de matière sèche
- β-glucosidases NOVOZYM 188, 25 CBU par gramme de matière sèche

Les solutions sont ensuite agitées à 400 tr/min à 50°C, toujours dans les bains marie à sec, et des prélèvements sont effectués au bout de 1 h, 4 h, 7 h et 24 h. Ces prélèvements sont placés dans des tubes à centrifuger et rapidement placés 10 minutes dans une huile à 103°C pour neutraliser l'activité enzymatique. Les tubes à centrifuger sont stockés dans un réfrigérateur à 4°C en attendant la mesure en glucose. Ils sont ensuite dilués par 5 avec de l'eau distillée avant d'être dosés à l'aide de l'appareillage analyseur Analox GL6, appelé glucostat, qui mesure par dosage enzymatique la concentration en glucose dans des solutions aqueuses.
Les résultats sont présentés dans le tableau 1.

### Exemple 4: Comparatif hydrolyse enzymatique:

La cellulose Avicel PH 101 (Sigma-Aldrich) est placé dans des flacons Schott de 100 ml. 5 ml de tampon acétate et 10 ml d'une solution 1% poids de NaN₃ dans de l'eau sont ajoutés puis la solution est complétée jusqu'à 100 g avec de l'eau distillée. Cette solution est ensuite laissée une nuit à 50°C pour "activation" sous une agitation de 550 tr/min dans un bain marie à sec STEM. On ajoute ensuite à la solution des quantités connues d'enzyme:
- Cellulases XL508, 10FPU par gramme de matière sèche
- β-glucosidases NOVOZYM 188, 25 CBU par gramme de matière sèche

Les solutions sont ensuite agitées à 400 tr/min à 50°C, toujours dans les bains marie à sec, et des prélèvements sont effectués au bout de 1 h, 4 h, 7 h et 24 h. Ces prélèvements sont placés dans des tubes à centrifuger et rapidement placés 10 minutes dans une huile à 103°C pour neutraliser l'activité enzymatique. Les tubes à centrifuger sont stockés dans un réfrigérateur à 4°C en attendant la mesure en glucose. Ils sont ensuite dilués par 5 avec de l'eau distillée avant d'être dosés à l'aide de l'appareillage glucostat qui mesure la concentration en glucose dans des solutions aqueuses.
Les résultats sont présentés dans le tableau 1.

**Tableau 1: Concentration en glucose obtenu après hydrolyse enzymatique**

| | Concentration en glucose dans la solution (mg/dl) | | | |
|---|---|---|---|---|
| | 1h | 4h | 7h | 24h |
| Prétraitement AMI-DEPS (selon exemple 3) | 152,0 | 358,5 | 471,0 | 694,0 |
| Cellulose native (selon exemple 4) | 112,8 | 248,0 | 334,0 | 608,0 |

Il s'agit de la concentration en glucose mesurée par dosage glucostat (concentration en glucose en mg/dl) après 1, 4, 7 et 24h d'hydrolyse enzymatique pour la cellulose avicel native (non-traitée) et pour la cellulose prétraitée par l'allyl-3-méthyl-1-imidazolium (AMI-DEPS)

La concentration en glucose obtenue est nettement supérieure avec le prétraitement au liquide ionique, ainsi après 24h d'hydrolyse 608 mg/dl de glucose théorique est formé dans le cas de la cellulose non-traitée en comparaison avec la concentration de 694 mg/dl associée au prétraitement à l'AMI-DEPS.

## Revendications

1. Liquide ionique associant un cation organique Q⁺ à un anion thiophosphate, répondant à l'une des formules suivantes : 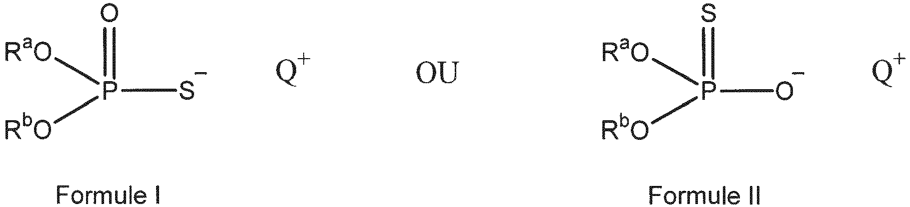 dans lesquelles
- R^{a} et R^{b}, identiques ou différents, représentent des radicaux hydrocarbyles ayant de 1 à 30 atomes de carbone,
- tout ou partie des hydrogènes des radicaux R^{a} et R^{b} pouvant être remplacés par des hétéroatomes, tels que des halogènes,
- les radicaux R^{a} et R^{b} portant éventuellement une ou plusieurs fonctions choisies parmi les fonctions -CO₂R, -C(O)R, -OR, -C(O)NRR', -C(O)N(R)NR'R", -NRR', -SR,-S(O)R, -S(O)₂R, -SO₃R, -CN, -N(R)P(O)R'R', -PRR', -P(O)RR', -P(OR)(OR'),-P(O)(OR)(OR') dans lesquelles R, R' et R", identiques ou différents, représentent chacun l'hydrogène ou des radicaux hydrocarbyles ayant de 1 à 30 atomes de carbone,
le cation Q+ étant choisi dans le groupe formé par le *N*-butylpyridinium, le *N-*éthylpyridinium, l'éthyl-3-méthyl-1-imidazolium, le butyl-3-méthyl-1-imidazolium, l'allyl-3-méthyl-1-imidazolium, le vinyl-3-méthyl-1-imidazolium, le vinyl-3-allyl-1-imidazolium, l'hexyl-3-méthyl-1-imidazolium, le butyl-3-diméthyl-1,2-imidazolium, le cation (hydroxy-2-éthyl)-1-méthyl-3-imidazolium, le cation (carboxy-2-éthyl)-1-méthyl-3-imidazolium, le diéthylpyrazolium, le *N*-butyl-*N*-méthylpyrrolidinium, le *N*-butyl-*N-*méthylmorpholinium, le tétrabutylphosphonium, le tributyl-tétradécyl-phosphonium.

2. Liquide ionique selon la revendication 1 **caractérisé en ce qu'**il est choisi dans le groupe constitué de diéthylthiophosphate d'allyl-3-méthyl-1-imidazolium, le diéthylthiophosphate de vinyl-3-méthyl-1-imidazolium, le diéthylthiophosphate d'éthyl-3-méthyl-1-imidazolium, le diéthylthiophosphate de butyl-3-méthyl-1-imidazolium, le diphénylthiophosphate d'allyl-3-méthyl-1-imidazolium, le diphénylthiophosphate de vinyl-3-méthyl-1-imidazolium, le diphénylthiophosphate d'éthyl-3-méthyl-1-imidazolium, le diphénylthiophosphate de butyl-3-méthyl-1-imidazolium, le dibenzylthiophosphate d'allyl-3-méthyl-1-imidazolium, le dibenzylthiophosphate de vinyl-3-méthyl-1-imidazolium, le dibenzylthiophosphate d'éthyl-3-méthyl-1-imidazolium, le dibenzylthiophosphate de butyl-3-méthyl-1-imidazolium.

3. Procédé de préparation d'un liquide ionique selon l'une des revendications 1 ou 2 dans lequel on réalise une réaction de métathèse d'ions entre :
- un sel de formule générale Q⁺A⁻, avec Q⁺ un cation tel que défini précédemment et A⁻ un anion, organique ou inorganique, et
- un sel de thiophosphate de formule générale: 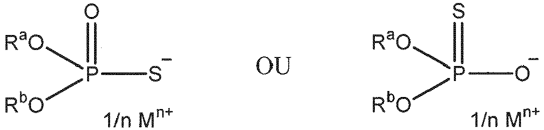 dans laquelle
- R^{a} et R^{b}, identiques ou différents, représentent des radicaux hydrocarbyles ayant de 1 à 30 atomes de carbone,
- tout ou partie des hydrogènes des radicaux R^{a} et R^{b} pouvant être remplacés par des hétéroatomes, tels que des halogènes,
- les radicaux R^{a} et R^{b} portant éventuellement une ou plusieurs fonctions choisies parmi les fonctions -CO₂R, -C(O)R, -OR, -C(O)NRR', -C(O)N(R)NR'R", -NRR', -SR,-S(O)R, -S(O)₂R, -SO₃R, -CN, -N(R)P(O)R'R', -PRR', -P(O)RR', -P(OR)(OR'),-P(O)(OR)(OR') dans lesquelles R, R' et R", identiques ou différents, représentent chacun l'hydrogène ou des radicaux hydrocarbyles ayant de 1 à 30 atomes de carbone
et Mⁿ⁺ est un cation alcalin ou alcalino-terreux et n= 1 ou 2.

4. Procédé selon la revendication 3 dans lequel l'anion A⁻ est choisi parmi les anions chlorure, bromure, iodure, nitrate, formate ou acétate.

5. Procédé selon la revendication 3 ou 4 dans lequel le cation Mⁿ⁺ est choisi parmi les cations lithium, sodium, potassium, magnésium ou calcium.

6. Procédé de préparation d'un liquide ionique selon l'une des revendications 1 ou 2 dans lequel on met en oeuvre une réaction entre :
- un sel de formule générale Q⁺A⁻, avec Q⁺ un cation tel que défini précédemment et A⁻ un anion, organique ou inorganique, et
- un acide de formule générale: 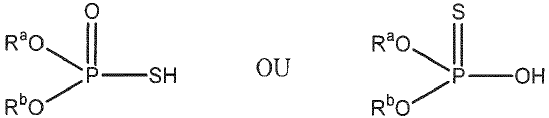 dans laquelle R^{a} et R^{b} sont définis comme précédemment.

7. Procédé de préparation d'un liquide ionique selon la revendication 6 dans lequel l'anion A⁻ est choisi alkylcarbonate, acétate, formate, hydroxyde.

8. Procédé selon l'une des revendications 3 à 7 **caractérisé en ce qu'**il est réalisé dans une plage de température comprise entre -78°C et 150°C, de façon préférée entre -20 °C et 80 °C, à l'air ou sous atmosphère de gaz inerte, en l'absence de solvant ou dans un solvant organique.

9. Procédé selon la revendication 8 dans lequel le solvant est choisi parmi les hydrocarbures tel que le pentane, l'hexane, l'heptane ou le cyclohexane, les solvants aromatiques tels que le benzène, le toluène, ou le xylène), les solvants chlorés tels que le chloroforme ou le dichlorométhane, les solvants oxygénés tels que le diéthyléther, le THF, le méthyl-THF, le dioxane, le diméthoxyéthane, les solvants azotés tels que l'acétonitrile, le DMF, le NMP ou la pyridine.

10. Utilisation du liquide ionique selon l'une des revendications 1 ou 2 ou préparé selon l'une des revendications 3 à 9 comme solvant ou catalyseur.

11. Utilisation selon la revendication 10 dans le traitement et/ou la solubilisation de biomasse lignocellulosique.

12. Utilisation selon la revendication 10 dans la production de bioproduits et biocarburants issus de matériaux riches en cellulose, tel que le 5-hydroxyméthylfurfural (HMF), le sorbitol, le diméthylfurane , l'acide lévulinique ou encore les esters et éthers d'HMF.

## Patentansprüche

1. Ionische Flüssigkeit, die ein organisches Kation Q⁺ mit einem Thiophosphat-Anion verbinden, entsprechend einer der folgenden Formeln: 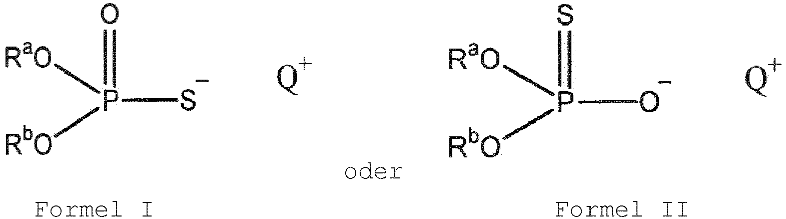 Wobei
- R^{a} und R^{b} identisch oder unterschiedlich sind und Kohlenwasserstoffgruppen mit 1 bis 30 Kohlenstoffatomen darstellen,
- die Gesamtheit oder ein Teil der Wasserstoffe der Gruppen R^{a} und R^{b} durch Heteroatome, wie Halogene, ersetzt sein können,
- die Gruppen R^{a} und R^{b} eventuell eine oder mehrere Funktionen tragen, die unter den Funktionen-CO₂R, -C(0)R, -OR, -C(O)NRR', -C(O)N(R)NR'R",-NRR', -SR, -S(O)R, -S(O₂)R, -SO₃R, -CN,-N(R)P(O)R'R', -PRR', -P(O)RR', -P(OR)(OR'),-P(O)(OR)(OR') ausgewählt sind, die jeden Wasserstoff oder Kohlenwasserstoffgruppen mit 1 bis 30 Kohlenstoffatomen darstellen,
wobei das Kation Q⁺ aus der Gruppe ausgewählt ist, gebildet durch N-Butylpyridinium, N-Ethylpyridinium, Ethyl-3-Methyl-1-Imidazolium, Vinyl-3-Methyl-1-Imidazolium, Vinyl-3-Allyl-1-Imidazolium, Hexyl-3-Methyl-1-Imidazolium, Butyl-3-Dimethyl-1,2-Imidazolium, dem Kation (Hydroxy-2-Ethyl)-1-Methyl-3-Imidazolium, dem Kation (Carboxy-2-Ethyl)-1-Methyl-3-Imidazolium, Diethylpyrazolium, N-Butyl-N-Methylpyrrolidinium, N-Butyl-N-Methylmorpholinium, Tetrabutylphosphonium, Tributyl-Tetradecyl-Phosphonium.

2. Ionische Flüssigkeit nach Anspruch 1, **dadurch gekennzeichnet, dass** sie gewählt ist aus der Gruppe gebildet durch, Diethylthiophosphat von Allyl-3-Methyl-1-Imidazolium, Diethylthiophosphat von Vinyl-3-Methyl-1-Imidazolium, Diethylthiophosphat von Ethyl-3-Methyl-1-Imidazolium, Diethylthiophosphat von Butyl-3-Methyl-1-Imidazolium, Diphenylthiophosphat von Allyl-3-Methyl-1-Imidazolium, Diphenylthiophosphat von Vinyl-3-Methyl-1-Imidazolium, Diphenylthiophosphat von Ethyl-3-Methyl-1-Imidazolium, Diphenylthiophosphat von Butyl-3-Methyl-1-Imidazolium, Dibenzylthiophosphat von Allyl-3-Methyl-1-Imidazolium, Dibenzylthiophosphat von Vinyl-3-Methyl-1-Imidazolium, Dibenzylthiophosphat von Ethyl-3-Methyl-1-Imidazolium, Dibenzylthiophosphat von Butyl-3-Methyl-1-Imidazolium.

3. Verfahren zur Herstellung einer ionischen Flüssigkeit nach einem der Ansprüche 1 oder 2, bei dem eine Ionenmetathese-Reaktion durchgeführt wird zwischen:
- einem Salz mit der allgemeinen Formel Q⁺A⁻, wobei Q⁺ ein Kation, wie vorher definiert, und A⁻ ein organisches oder anorganisches Anion ist, und
- einem Thiophosphat-Salz mit der allgemeinen Formel: 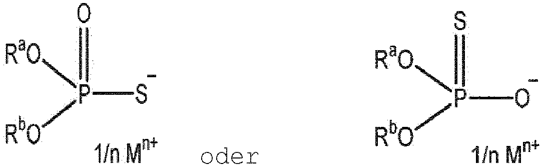 Wobei
- R^{a} und R^{b} identisch oder unterschiedlich sind und Kohlenwasserstoffgruppen mit 1 bis 30 Kohlenstoffatomen darstellen,
- die Gesamtheit oder ein Teil der Wasserstoffe der Radikale R^{a} und R^{b} durch Heteroatome, wie Halogene, ersetzt sein können,
- die Gruppen R^{a} und R^{b} gegebenenfalls eine oder mehrere Funktionen tragen, die unter den Funktionen -CO₂R, -C(O)R, -OR, -C(O)NRR',-C(O)N(R)NR'R", -NRR', -SR, -S(O)R, -S(O)2R,-SO₃R, -CN, -N(R)P(O)R'R', -PRR', -P(O)RR',-P(OR)(OR'), -P(O)(OR)(OR') ausgewählt sind, die jeden Wasserstoff oder Kohlenwasserstoffgruppe mit 1 bis 30 Kohlenstoffatomen darstellen,
- und Mⁿ⁺ ein alkalisches oder erdalkalisches Kation ist und n = 1 oder 2.

4. Verfahren nach Anspruch 3, bei dem das Anion A⁻ unter den Anionen Chlorid, Bromid, Iodid, Nitrat, Format oder Acetat ausgewählt ist.

5. Verfahren nach Anspruch 3 oder 4, bei dem das Kation Mⁿ⁺ unter den Kationen Lithium, Natrium, Kalium, Magnesium oder Kalzium ausgewählt ist.

6. Verfahren zur Herstellung einer ionischen Flüssigkeit nach einem der Ansprüche 1 oder 2, bei dem eine Reaktion durchgeführt wird zwischen:
- einem Salz mit der allgemeinen Formel Q⁺A⁻, wobei Q⁺ ein Kation, wie vorher definiert, und A⁻ ein organisches oder anorganisches Anion ist, und
- einer Säure mit der allgemeinen Formel: 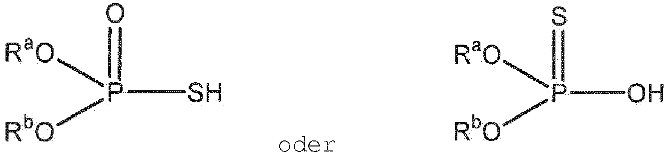 wobei R^{a} und R^{b} wie vorher definiert sind.

7. Verfahren zur Herstellung einer ionischen Flüssigkeit nach Anspruch 6, bei der das Anion A⁻ Alkylcarbonat, Acetat, Format, Hydroxid gewählt wird.

8. Verfahren nach einem der Ansprüche 3 bis 7, **dadurch gekennzeichnet, dass** es in einem Temperaturbereich zwischen -78°C und 150°C, auf bevorzugte Weise zwischen -20°C und 80°C, an der Luft oder unter Inertgasatmosphäre ohne Lösungsmittel oder in einem organischen Lösungsmittel durchgeführt wird.

9. Verfahren nach Anspruch 8, bei dem das Lösungsmittel unter den Kohlenwasserstoffen, wie Pentan, Hexan, Heptan oder Zyklohexan, den aromatischen Lösungsmitteln, wie Benzol, Toluol oder Xylol, den chlorhaltigen Lösungsmitteln, wie Chloroform oder Dichlormethan, den sauerstoffhaltigen Lösungsmitteln, wie Diethylether, THF, Methyl-THF, Dioxan, Dimethoxyethan, den stickstoffhaltigen Lösungsmitteln, wie Acetonitril, DMF, NMP oder Pyridin, ausgewählt ist.

10. Verwendung der ionischen Flüssigkeit nach einem der Ansprüche 1 oder 2 oder hergestellt nach einem der Ansprüche 3 bis 9 als Lösungsmittel oder Katalysator.

11. Verwendung nach Anspruch 10 bei der Behandlung und/oder Solubilisierung von lignozellulärer Biomasse.

12. Verwendung nach Anspruch 10 bei der Herstellung von Bioprodukten und Biokraftstoffen, die von an Zellulose reichen Stoffen stammen, wie 5-Hydroxymethylfurfural (HMF), Sorbitol, Dimethylfuran, Lävulinsäure oder auch die Ester und Ether von HMF.

## Claims

1. An ionic liquid associating an organic cation Q⁺ with a thiophosphate anion corresponding to one of the following formulae: 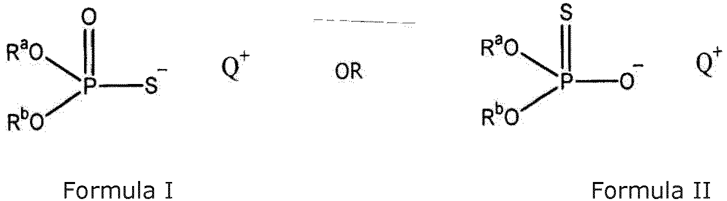 in which
- R^{a} and R^{b} which are identical or different represent hydrocarbyl radicals having from 1 to 30 carbon atoms,
- wherein all or part of the hydrogens of the radicals R^{a} and R^{b} can be replaced by heteroatoms such as halogens,
- the radicals R^{a} and R^{b} possibly bearing one or more functions selected from the functions -CO₂R, -C(O)R, -OR, -C(O)NRR',-C(O)N(R)NR'R", -NRR', -SR, -S(O)R, -S(O)₂R, -SO₃R, -CN, -N(R)P(O)R'R',-PRR', -P(O)RR', -P(OR)(OR'), -P(O)(OR)(OR') in which R, R' and R" which are identical or different each represent hydrogen or hydrocarbyl radicals having from 1 to 30 carbon atoms,
the cation Q+ being selected from the group formed by N-butylpyridinium, N-ethylpyridinium, 1-ethyl-3-methyl imidazolium, 1-butyl-3-methyl imidazolium, 1-ally-3-methyl-imidazolium, 1-vinyl-3-methyl imidazolium, 1-vinyl-3-allyl imidazolium, 1-hexyl-3-methyl imidazolium, 1,2-butyl-3-dimethyl imidazolium, the cation (hydroxyl-2-ethyl)-1-methyl-3-imidazolium, the cation (carboxy-2-ethyl)-1-methyl-3-imidazolium, diethylpyrazolium, N-butyl-N-methylpyrrolidinium, N-butyl-N-methyl-morpholinium, tetrabutylphosphonium and tributyltetradecylphosphonium.

2. An ionic liquid according to claim 1 **characterised in that** it is selected from the group constituting of 1-ally-3-methyl-imidazolium diethylthiophosphate, 1-vinyl-3-methyl imidazolium diethylthiophosphate, 1-ethyl-3-methyl imidazolium diethylthiophosphate, 1-butyl-3-methyl imidazolium diethylthiophosphate, 1-ally-3-methyl imidazolium diphenylthiophosphate, 1-vinyl-3-methyl imidazolium diphenylthio-phosphate, 1-ethyl-3-methyl imidazolium diphenylthiophosphate, 1-butyl-3-methyl imidazolium diphenylthiophosphate, 1-allyl-3-methyl imidazolium dibenzylthiophosphate, 1-vinyl-3-methyl imidazolium dibenzylthio-phosphate, 1-ethyl-3-methyl imidazolium dibenzylthiophosphate, and 1-butyl-3-methyl imidazolium dibenzylthiophosphate.

3. A process for the preparation of an ionic liquid according to one of claims 1 and 2 in which a metathesis reaction of ions is effected between:
- a salt of the general formula Q⁺A⁻, with Q⁺ being a cation as defined hereinbefore and A⁻ being an organic or inorganic anion, and
- a thiophosphate salt of the general formula: 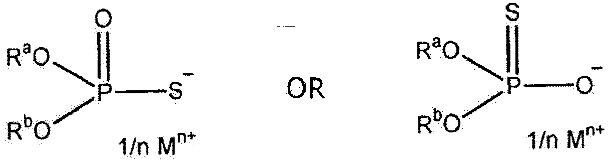 in which
- R^{a} and R^{b} which are identical or different represent hydrocarbyl radicals having from 1 to 30 carbon atoms,
- wherein all or part of the hydrogens of the radicals R^{a} and R^{b} can be replaced by heteroatoms such as halogens,
- the radicals R^{a} and R^{b} possibly bearing one or more functions selected from the functions -CO₂R, -C(O)R, -OR, -C(O)NRR',-C(O)N(R)NR'R", -NRR', -SR, -S(O)R, -S(O)₂R, -SO₃R, -CN, -N(R)P(O)R'R',-PRR', -P(O)RR', -P(OR)(OR'), -P(O)(OR)(OR') in which R, R' and R" which are identical or different each represent hydrogen or hydrocarbyl radicals having from 1 to 30 carbon atoms, and
Mⁿ⁺ is an alkali or alkaline earth metal cation and n = 1 or 2.

4. A process according to claim 3 wherein the anion A⁻ is selected from the anions chloride, bromide, iodide, nitrate, formate or acetate.

5. A process according to claim 3 or claim 4 wherein the cation Mⁿ⁺ is selected from the cations lithium, sodium, potassium, magnesium or calcium.

6. A process for the preparation of an ionic liquid according to one of claims 1 and 2 in which a reaction is performed between:
- a salt of the general formula Q⁺A⁻, with Q⁺ being a cation as defined hereinbefore and A⁻ being an organic or inorganic anion, and
- an acid of the general formula: 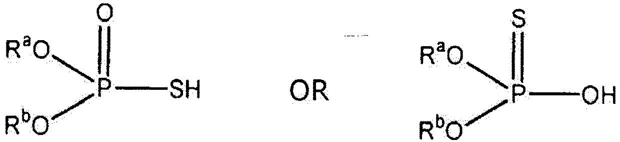 in which R^{a} and R^{b} are defined as hereinbefore.

7. A process for the preparation of an ionic liquid according to claim 6 in which the anion A⁻ is selected from alkylcarbonate, acetate, formate and hydroxide.

8. A process according to one of claims 3 to 7 **characterised in that** it is carried out in a temperature range of between -78°C and 150°C, preferably between -20°C and 80°C, in air or in an inert gas atmosphere, in the absence of solvent or in an organic solvent.

9. A process according to claim 8 in which the solvent is selected from hydrocarbons such as pentane, hexane, heptane or cyclohexane, aromatic solvents such as benzene, toluene or xylene), chlorinated solvents such as chloroform or dichloromethane, oxygenated solvents such as diethylether, THF, methyl-THF, dioxane, dimethoxyethane, and nitrogenous solvents such as acetonitrile, DMF, NMP or pyridine.

10. Use of the ionic liquid according to one of claims 1 and 2 or prepared according to one of claims 3 to 9 as a solvent or catalyst.

11. Use according to claim 10 in the treatment and/or solubilisation of lignocellulosic biomass.

12. Use according to claim 10 in the production of bioproducts and biofuels from cellulose-rich materials such as 5-hydroxymethylfurfural (HMF), sorbitol, dimethylfuran, levulinic acid or esters and ethers of HMF.
